(19) Europäisches Patentamt
European Patent Office
Office européen des brevets

(11) Publication number: **0 323 209 B1**

(12) **EUROPEAN PATENT SPECIFICATION**

(45) Date of publication of patent specification: **16.02.94**

(51) Int. Cl.[5]: **C11D 17/00**, C11D 3/37, C11D 3/12

(21) Application number: **88312341.6**

(22) Date of filing: **28.12.88**

(54) **Detergent compositions.**

(30) Priority: **30.12.87 US 139490**
**30.12.87 US 139355**
**30.12.87 US 139357**
**30.12.87 US 139492**

(43) Date of publication of application:
**05.07.89 Bulletin 89/27**

(45) Publication of the grant of the patent:
**16.02.94 Bulletin 94/07**

(84) Designated Contracting States:
**CH DE ES FR GB IT LI NL SE**

(56) References cited:
**EP-A- 0 120 533**
**EP-A- 0 295 093**
**WO-A-86/05199**
**CA-A- 1 031 229**
**GB-A- 1 471 406**
**GB-A- 2 163 447**
**US-A- 4 215 004**
**US-A- 4 228 048**
**US-A- 4 362 715**

(73) Proprietor: **UNILEVER PLC**
**Unilever House**
**Blackfriars**
**P.O. Box 68**
**London EC4P 4BO(GB)**
(84) Designated Contracting States:
**GB**

(73) Proprietor: **UNILEVER N.V.**
**Weena 455**
**NL-3013 AL Rotterdam(NL)**
(84) Designated Contracting States:
**CH DE ES FR IT LI NL SE**

(72) Inventor: **Corring, Robert**
**69 Miami Trail**
**Rockaway Township New Jersey 08766(US)**
Inventor: **Steyn, Peter Lucian**
**34 Marshall Street**
**West Caldwell New Jersey 07006(US)**
Inventor: **Gabriel, Robert**
**141 Oakview Avenue**
**Maplewood New Jersey 07040(US)**

(74) Representative: **Tan, Bian An, Ir. et al**
**Unilever N.V.**
**Patent Division**
**P.O. Box 137**
**NL-3130 AC Vlaardingen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 323 209 B1

## Description

The invention relates to a detergent composition in gel form useful for a wide variety of cleaning purposes and of special application for cleaning dishes in an automatic dishwasher.

Automatic dishwashing detergents for home use have traditionally been in powder or granulate form. More recently, the marketplace has seen the advent of liquid forms of automatic dishwashing products. Liquids have advantages over powders in their convenience of dispensing or dosing, their enhanced solubility, absence of lump formation or "caking" during storage, and absence of dustiness associated with the powder form.

Since automatic dishwashing machines contain a dispenser cup normally intended for powders, chemists have been challenged in formulating a liquid product of appropriate rheological properties.

Firstly, the composition must be a uniform mixture to deliver an optimum combination of active ingredients to the wash with each dose. Thus, the liquid must possess physical stability against syneresis or physical separation of its active components during storage.

Secondly, a liquid product must be compatible with automatic dishwashing equipment presently available to the consumer. Home dishwashers are fitted with a closed cup to house detergent through several cycles preliminary to the wash cycle. Cups in these machines do not seal tightly and do not adequately retain liquids of low viscosity. Excessive leakage leads to underdosing in the wash cycle. Performance may be adversely affected. Consequently, any liquid product must possess high viscosity to be effectively retained in the cup and avoid leakage into cycles preceding that of the wash.

Conversely, there are situations where the product should have low viscosity. A low viscosity is desirable for easy dispensing of product from its bottle. Thixotropic liquids address the foregoing dilemma by maintaining high viscosity for storage but reverting to lower viscosity under influence of applied shear. Thixotropy is shear thinning behavior that is time dependent in both its decrease in viscosity under applied shear and its regain of viscosity after cessation of shearing.

The earliest approaches to these problems involved the use of clays to modify viscosity. Typical of this technology are the compositions disclosed in U.S. Patent 4,116,849, U.S. Patent 4,431,559, GB 2 116 199A, GB 2 140 450A and CA-A-1 031 229. Some patents such as U.S. 4,511,487 and U.S. 4,512,908 have singled out hectorite as a particularly efficient thickener. There has also been reported in U.S. Patent 3,558,496 the advantage of combining a negatively charged clay such as hectorite with a positively charged clay such as alumina clay.

GB 2 176 495A suggests the use of polyvalent metal salts of long chain fatty acids, such as aluminum or zinc stearate, as stabilizers against phase separation in a clay laiden liquid composition. Another method of improving phase stability in thixotropic liquids is reported in GB 2 163 448A. This patent suggests inclusion of a limited amount of a water-soluble potassium salt to achieve a potassium: sodium weight ratio of about 0.04 to 0.5. Relatively large crystals are said to be inhibited from forming when potassium is present thereby resulting in greater stability against separation on ageing. U.S. Patent 3,720,621 reports a further useful property of including some potassium salt within a sodium tripolyphosphate liquid composition. Here the presence of potassium allows the amount of sodium tripolyphosphate included within the aqueous detergent to attain a considerably higher solubility than found in the absence of potassium.

Although generally acceptable, clay structured liquids have a number of disadvantages. Montmorillonite clays, even in the presence of stabilizing agents, are sensitive to ionic strength. They lose their liquid structuring efficiency at the high electrolyte levels normally present in autodish liquid detergents. Clays tend to collapse onto themselves, or flocculate under these conditions. If this collapse occurs to any large extent during prolonged storage, the liquid will lose its physical stability, suffer syneresis and/or settling of solids. Collection of solids at the bottom of the container can lead to the formation of paste-like plugs which are difficult to dispense.

Attapulgite clay particles suspended in liquids tend to scatter light. Any large amount of these clay particles will thus impart a muddy dull color to the liquid. Furthermore, clays, being insoluble minerals, can adversely affect glass appearance. Deposition of clay onto the surface of glassware has been known to lead to spotting and filming.

Another problem of suspended solids in prior art liquids is that they are subject to recrystallization during storage periods. Through a process of Ostwald ripening, the solids can redistribute themselves in terms of number and size of crystals. These changes can cause a drastic change in rheology of the liquid over time. Poor stability and/or cup retention result.

Many polymers are known for their thickening properties. Within the machine dishwashing art, polyacrylic acid type polymers have been included as an important component but not necessarily to function as a thickener. Thus, U.S. Patent 3,579,455 discusses what is evidently a powdered dishwashing

detergent utilizing sodium polyacrylate as an anti-spotting/streaking agent and hardness precipitator. Polyacrylate has also been incorporated into thixotropic liquids that have been primarily thickened with powdered clay. GB 2 163 447A and GB 2 164 350A contain such clay-sodium polyacrylate systems and suggest that the polymer provides improved protection to the overglaze layer of fine china. Less filming on glassware was also noted.

Use of polymers for gel-formation in liquid detergent compositions was suggested in U.S. Patent 3,060,124. Apparently, cross-linked vinyl polymers are primarily suitable. Hydrolyzed polyacrylonitrile cross-linked with formaldehyde was found particularly effective at stabilizing the gels against separation. U.S. Patents 4,228,048, and 4,215,004, and GB Patent 1 471 406 and EP-A-0 120 533 illustrate the use of polyallyl sucrose cross-linked polyacrylates, commercially available under the trademark Carbopol®, as a thickener for liquid cleaning and bleaching concentrates. Japanese Laid Open Patents 59-36198 (Kao Soap) and 59-36200 (Kao Soap) further illustrate the use of polyacrylate cross-linked with compounds such as allylated pentaerythritol. These thickened formulas are used to suspend water-insoluble abrasives such as silicone dioxide and aluminum oxide.

Although the aforementioned polymer systems do provide some measure of thickening and phase stabilization, they are frequently not fully adequate at such functions, especially where there is a high level of electrolyte present. Systems are required exhibiting improved stability against phase separation at high electrolyte level and having improved rheological properties. With regard to rheology, the composition must not substantially leak from the cup of an automatic dishwasher, but at the same time be sufficiently shearing to allow flow out of its container.

There has also been a search for more aesthetically pleasing product forms. Clay structurants cream the carrier liquid resulting in an opaque product. Many polymers also impart opaque properties. Clear compositions would, by contrast, be more aesthetically pleasing to the consumer.

We have appreciated that liquids including all those of the aforementioned art have another characteristic where improvement would be desirable. Subsequent to pouring, the mouth of the pouring container will retain flow cut-off product droplets. Normally, these droplets will travel from the lip downward along the outside of the container. Consumers do not like the resulting mess. Some containers have been designed with special pour spouts to prevent this problem. The spouts are, however, quite expensive and not normally used for small-sized containers. It would therefore be desirable to obtain a product inherently having non-drip properties.

Accordingly, it is an object of the present invention to obtain a composition in gel form readily flowable from its container but, nevertheless, having rigidity when not subjected to shearing forces. With particular respect to automatic dishwashing compositions, it is an object of the present invention to provide a gel product that can readily be dispensed from its bottle but, once placed in a retaining cup of an automatic dishwasher will have sufficient thickness not to leak from the cup before dispensing.

A further object of the present invention is to provide a gel cleaning composition pourable from a container similar to ordinary liquids but having a recoil elasticity rendering the composition dripless.

Accordingly the invention provides an aqueous cleaning composition in a gel form having a viscosity on a Haake Rotovisco RV-100 Viscometer at 25 °C under 5 $sec^{-1}$ shear of from 1,000 to 20,000 cps and under 21 $sec^{-1}$ shear of from 200 to 5,000 cps, and a pH range from 11 to 13, which is characterized in that it comprises:

(i) from 0.1 to 10% by weight of a thickener that is a cross-linked polycarboxylic polymer; and

(ii) from 0.01 to 10% by weight of a trivalent metal containing material other than aluminosilicate,

said composition having a steady state viscoelastic deformation compliance $J_e°$ value greater than 0.01 $m^2$/Newton.

Preferred forms of this invention display some or all of the following additional advantages:-

clarity,

good storage stability so as to avoid phase separation,

reduced spotting and filming on glassware,

avoidance of problems associated with clay as sole structurant.

The aqueous cleaning compositions of the present invention have several properties which are unusual and surprising. Unlike known gel compositions, the present material has an elastic nature rendering the material non-dripping. When tilting a container upright again after pouring, the discharging gel exhibits a memory, recoiling back into the container without leaving any drop of liquid around the container mouth. The effect is somewhat akin to the action of a yo-yo. Gel elasticity is believed to arise from strong intermolecular entwinning which does not seem to occur in other systems. A physical measure of this elasticity or recoil is $J_e°$, the steady state compliance value. $J_e°$ is derived from steady state viscoelastic deformation measurements performed through well known standard techniques (see J. Ferry, "Viscoelastic

Properties of Polymers", Third Edition, John Wiley & Sons, New York, 1980). $J_e°$ reflects the elastic deformation and/or energy stored in the elastic components of a fluid during steady flow. This value identifies the extent to which a fluid rebounds when stress is removed. Rebounding or recoil is a property associated with visual perception of elasticity. The $J_e°$ value should be greater than 0.01 $meters^2$/Newton, preferably greater than 0.02 $meter^2$/Newton. The range may extend up to 0.10 $meter^2$/Newton and optimally is a range from 0.025 to 0.6 $meter^2$/Newton.

Gel compositions of this invention must also have acceptable flowability from a container but, when at rest, must be relatively non-flowing. The non-flowing property is important in such areas such as automatic dishwashing detergents. When such a detergent is placed in an automatic dishwashing dispenser cup, the detergent composition should have sufficient structural integrity not to rapidly flow out of the dispenser cup. Thus, gel compositions of this invention should possess under the very low shear conditions of 5 $sec^{-1}$ at 25°C, a viscosity of from 1,000 to 20,000 cps, preferably from 1,500 to 10,000 cps, optimally between 3,000 and 7,000 cps. Under flow conditions represented by the shear rate of 21 $sec^{-1}$ at 25°C, the viscosity should range from 200 to 5,000 cps, preferably from 800 to 4,000 cps, optimally from 900 to 2500 cps. The aforementioned viscosities are measured on a Haake Rotovisco RV-100 Viscometer. A pH range for these liquids varies from 11 to 13.

Another unusual property that certain embodiments of the present invention may possess is that of clarity or near transparency. The term "clear" as used in this specification is intended to connote its usual dictionary definition. Thus, a clear composition allows ready viewing of objects behind it. By contrast, a translucent composition although allowing light to pass through, causes light to be so scattered as by a very small portion of crystals or insolubles, thus it will be impossible to clearly identify objects behind the translucent material. Within the context of this invention, the composition is deemed to be clear if the maximum transmittance of light through a sample 2 cm thick is at least 10%, preferably at least 20%, optimally greater than 50%. A gel is deemed translucent if the maximum transmittance of such light through the sample is between 5% and 10%. Finally, a gel is deemed opaque if the maximum transmittance of light is below 5%. A suitable test for measuring transmittance is to place a sample of the aforestated thickness in the light path probe of a Brinkmann PC 800, Colorimeter fitted with a 470 nm filter. Distilled water is considered a baseline for 100% transmittance.

We have found that a gel with the aforedescribed unique recoil properties can be obtained by use of a cross-linked polycarboxylate polymer and another material, as will be explained below. Moreover, there preferably should be present a structuring chelant.

Polycarboxylic thickening polymers in aqueous media are known to tolerate, without phase separation, modest electrolyte levels in such products as liquid automatic dishwashing detergents. Problems of phase separation can be expected when these modest electrolyte levels are substantially increased by the addition of further salts. However, we have found that certain cross-linked polycarboxylic polymers will impart a reasonably high viscosity to liquids even in the presence of high levels of salts.

Preferred polycarboxylic polymer is a polycarboxylic polymer that has been interpolymerized with a multi-vinyl or multi-allylic functionalized cross-linking agent. Preferably, the polycarboxylic polymer is interpolymerized with a polyalkenyl polyether of a polyhydric compound. The polyhydric compound should preferably have at least 4 carbons and 3 hydroxy groups. These thickeners are described in U.S. Patent 2,798,053 and U.S. Patent 4,130,501. Preferably the thickeners are water dispersible copolymers of an alpha-beta monoolefinically unsaturated lower aliphatic carboxylic acid cross-linked with a polyether of a polyol. The polyol may be selected from the group consisting of oligosaccharides, reduced derivatives thereof in which the carbonyl group is converted to an alcohol group, and pentaerythritol. The hydroxy groups of said polyol may be etherified with allyl groups, said polyol having at least two allyl groups per polyol molecule. A suitable copolymer is one of acrylic acid with low percentages (0.71 to 1.5%) of polyallyl sucrose.

Molecular weights of the cross-linked polymer may range from 500,000 up to 10,000,000, preferably between 500,000 and 2,000,000, optimally about 1,250,000. Examples of commercially available cross-linked polymers based upon allyl sucrose modified polyacrylic acid are the Carbopol® resins manufactured by the B.F. Goodrich Chemical Company. These materials include Carbopol 941® (m.w. 1,250,000), Carbopol 934® (m.w. 3,000,000) and Carbopol 940® (m.w. 4,000,000). Most preferred is Carbopol 941® which gives the best structuring and clarity.

The polymeric thickener used in this invention is present in an amount from 0.1 to 10%, preferably from 0.5 to 2%, optimally between 0.7 and 1.5% by weight of the composition.

We have found that high viscosity conferred by polymeric thickener is not, on its own, sufficient for certain product systems. For instance, automatic dishwashing products require an adequate level of product retention within the cup of a dishwashing machine. For such product applications, it has been found

desirable to include an additional material that will interact with the cross-linked polycarboxylic polymers to substantially improve product rheology.

This additional material to be included, as co-structurant together with the polymeric thickener, is a material containing a trivalent metal. Most effective are those materials containing aluminum, especially aluminum salts or aluminum oxides. Among the inorganic aluminum salts that have been found useful are those with counterions selected from sulfate, chloride, phosphate, nitrate, chlorhydroxide, bromide, carbonate and/or fluoroborate, or a mixture thereof. Alumina is however the most effective source of aluminum. A lost preferred form of this material is boehmite, a crystalline phase of aluminum oxyhydroxide. Especially desirable is a semi-crystalline phase commonly referred to as pseudoboehmite. Aluminosilicates were found not to be effective co-structurants and, for purposes of this invention, are excluded as the trivalent metal ion source. Of course, aluminosilicates (e.g. zeolites) might be present for other purposes, such as for calcium hardness sequestration, in the gel compositions, especially where clarity of the fully formulated product is unnecessary.

Trivalent metal containing material will be present in an amount from 0.01 up to 10%, preferably from 0.1 to 4%, optimally from 0.1 to 2% by weight of the composition.

A further material, which can be included, as co-structurant together with the polymeric thickener and the trivalent metal containing material, is a clay. Remarkably small amounts of clay are needed to combine with the cross-linked polycarboxylic polymer to achieve the desired rheological properties. As a result, gels are obtained having increased clarity and higher capacity for suspending solids. The clay may be accompanied by a chelant as mentioned below.

The clays which have been found to be most suitable are those of the smectite variety. Within this group the preferred clays are the synthetic and natural hectorites which are magnesium silicates. If a natural clay is used, it is preferably a purified hectorite such as Macaloid R®, supplied by N.L. Industries. A typical analysis of Macaloid on a dry basis is 51.89% $SiO_2$, 22.07% MgO, 1.21% $Li_2O$, 3.08% $Na_2O$, 6.46% CaO, 0.32% $Fe_2O_3$, 0.77% $Al_2O_3$ and 2.07% F. Particularly preferred, however, are the synthetic hectorites such as those available under the names "Laponite®" sold by Laporte Industries, Ltd. Suitable grades of Laponite® are Laponite S®, Laponite RDS®, Laponite RD®, and most preferred Laponite XLS®. The latter is a synthetic hectorite in the form of platelets, and having the following typical analysis: 59.8% $SiO_2$, 27.2% MgO, 4.4% $Na_2O$, 0.8% $Li_2O$, and 6% tetrasodium pyrophosphate.

Normally, hectorite will be used in amounts from 0.005 up to 0.1% by weight of the composition. Preferably, the amount will range from 0.01 up to 0.05%, optimally about 0.02% by weight. Amounts of clay substantially beyond 0.1% will not materially improve structuring. There will, however, be certain disadvantages such as clay depositing and leaving a film upon the substrates to be cleaned.

Dependent upon the chosen polymer and co-structurant, it may be desirable to also include a water-soluble structuring chelant. A matrix formed through the interaction of cross-linked polymer aluminum compound, optionally hectorite, and structuring chelant affords a salt tolerant gel unaffected by the presence of alkaline sources, builder salts and other soluble ionic species. The system provides for complete solubility of the foregoing components; the matrix is a highly suspending one. Thereby is achieved the additional benefit of eliminating suspended solids, and the attendant settling-separation problems. If desired, significant amounts of light dispersing solids may nevertheless be included in the matrix. Translucent or opaque gels would then result.

Particularly suitable as water-soluble structuring chelant are salts of carbonate, pyrophosphate and mixtures of these two materials. For purposes of product clarity, it is preferable to select potassium as the counterion to the carbonate and/or pyrophosphate. Small amounts of sodium may, however, be tolerated. Thus, the molar ratio of potassium to sodium ion should preferably be greater than 1:1, and optimally greater than 4:1. Under situations where potassium carbonate and potassium pyrophosphate are both present, the relative ratio of these chelants will best be from 1:10 to 10:1, preferably from 1:4 to 4:1, optimally 1:4 to 1:1.5. The amount of chelant may range anywhere from 1% up to 60%, preferably between 5 and 40%, more preferably 15 and 35%, optimally between 25 and 30% by weight of the composition.

When the gel composition is used as an automatic dishwashing formulation, it will normally also contain an oxidizing agent. Traditionally, liquid dishwashing compositions have for this purpose utilized sodium hypochlorite because it is inexpensive. Other oxidizing agents may, however, be employed. For instance, it is also possible to utilize heterocyclic N-bromo and N-chloro imides such as trichlorocyanuric, tribromocyanuric, dibromo and dichlorocyanuric acids, and salts thereof with water solubilizing cations such as potassium and sodium. An example of a hydrated dichlorocyanurate acid is Clearon® CDB 56, a product manufactured by the Olin Corporation. The oxidizing material may be present in the mixture from 0.1 to 10%, with the most preferred range being from 0.1 to 2% by weight. Preferred concentrations will provide 0.2 to 1.5 weight % available chlorine.

Automatic dishwashing detergent compositions based upon this invention will generally also contain sodium or potassium silicate. This material is employed as a cleaning ingredient, source of alkalinity, metal corrosion inhibitor, and protector of glaze on china tableware. Especially effective is sodium silicate having a ratio of $SiO_2$:$Na_2O$ from 1.0 to 3.3, preferably from 2 to 3.2. The silicate may be used in the form of an aqueous liquor or a solid. It will be present from 0.1 to 25%, more preferably from 5 to 10% by weight of the composition.

Surfactants are desirably part of the aforementioned compositions. These surfactants should preferably be of the low-foaming type; foam interferes with the dishwasher cleaning action. Suitable surfactants may be selected from nonionic, anionic and amphoteric types and mixtures thereof. Nonionic surfactants can be broadly defined as compounds produced by the condensation of alkylene oxide groups with an organic hydrophobic material which may be aliphatic or alkyl aromatic in nature. The length of the hydrophilic or polyoxyalkylene radical which is condensed with any particular hydrophobic group can be readily adjusted to yield a water-soluble compound having the desired degree of balance between hydrophilic and hydrophobic elements. Illustrative, but not limiting examples, of the various chemical types as suitable nonionic surfactants include:

(a) polyoxyethylene or polyoxypropylene condensates of aliphatic carboxylic acid, whether linear- or branched-chain and unsaturated or saturated, containing from 8 to 18 carbon atoms in the aliphatic chain and incorporating from 5 to 50 ethylene oxide and/or propylene oxide units. Suitable carboxylic acids include "coconut" fatty acids (derived from coconut oil) which contain an average of about 12 carbon atoms, "tallow" fatty acids (derived from tallow-class fats) which contain an average of about 18 carbon atoms, palmitic acid, myristic acid, stearic acid and lauric acid.

(b) polyoxyethylene or polyoxypropylene condensates of aliphatic alcohols, whether linear- or branched-chain and unsaturated or saturated, containing from 6 to 24 carbon atoms and incorporating from 5 to 50 ethylene oxide and/or propylene oxide units. Suitable alcohols include the "coconut" fatty alcohol, "tallow" fatty alcohol, lauryl alcohol, myristyl alcohol and oleyl alcohol. Particularly preferred nonionic surfactant compounds in this category are the "Neodol" type products, a registered trademark of the Shell Chemical Company.

Included within this category are nonionic surfactants having the formula:

$$R\text{-}(CH_2\underset{\underset{R'}{|}}{C}HO)_x(CH_2CH_2O)_y(CH_2\underset{\underset{R''}{|}}{C}HO)_z\text{-}H$$

wherein R is a linear, alkyl hydrocarbon having an average of 6 to 10 carbon atoms, R′ and R″ are each linear alkyl hydrocarbons of 1 to 4 carbon atoms, x is an integer from 1 to 6, y is an integer from 4 to 15 and z is an integer from 4 to 25. A particularly preferred example of this category is Poly-Tergent SLF-18, a registered trademark of the Olin Corporation, New Haven, Conn. Poly-Tergent SLF-18 has a composition of the above formula where R is a $C_6$-$C_{10}$ linear alkyl mixture, R′ and R″ are methyl, x averages 3, y averages 12, and z averages 16.

(c) polyoxyethylene or polyoxypropylene condensates of alkyl phenols, whether linear- or branched-chain and unsaturated or saturated, containing from 6 to 12 carbon atoms and incorporating from 5 to 25 moles of ethylene oxide and/or propylene oxide.

(d) polyoxyethylene derivatives of sorbitan mono-, di-, and tri-fatty acid esters wherein the fatty acid component has between 12 and 24 carbon atoms. The preferred polyoxyethylene derivatives are of sorbitan monolaurate, sorbitan trilaurate, sorbitan monopalmitate, sorbitan tripalmitate, sorbitan monostearate, sorbitan monoisostearate, sorbitan tristearate, sorbitan monooleate, and sorbitan trioleate. The polyoxyethylene chains may contain between 4 and 30 ethylene oxide units, preferably about 20. The sorbitan ester derivatives contain 1, 2 or 3 polyoxyethylene chains dependent upon whether they are mono-, di- or tri-acid esters.

(e) polyoxyethylene-polyoxypropylene block copolymers having the formula:

$$HO(CH_2CH_2O)_a(CH(CH_3)CH_2O)_b(CH_2CH_2O)_cH$$

wherein a, b and c are integers reflecting the respective polyethylene oxide and polypropylene oxide blocks of said polymer. The polyoxyethylene component of the block polymer constitutes at least about 40% of the block polymer. The material preferably has a molecular weight of between 2,000 and 10,000,

more preferably from 3,000 to 6,000. These materials are well known in the art. They are available under the trademark "Pluronics", a product of the BASF-Wyandotte Corporation.

Low foaming anionic surfactants are also very useful for this invention, especially when combined with effective defoaming materials. Anionics are desirable because they are more stable towards hypochlorite than the nonionic type. Illustrative of this category are alkyl diphenyloxide sulfonate, alkyl naphthalene sulfonate, sodium 2-acetamidohexadecane sulfonate and nonionic alkoxylates having a sodium alkylene carboxylate moiety linked to a terminal hydroxy group of the nonionic through an ether bond.

Surfactants will usually be present in an amount from 0.1 to 25%, preferably from 0.15 to 5%, optimally from 0.2 to 3% by weight of the composition.

A particularly preferred type of surfactant for incorporation into compositions of the present invention is alkyl polyglycosides. We have discovered that these give good cleaning while maintaining clarity of the compositions: many other surfactants fail to give clear compositions.

The preferred alkyl glycosides have the formula

$$RO(R'O)_y(Z)_x$$

wherein R is a monovalent organic radical (e.g., a monovalent saturated aliphatic, unsaturated aliphatic or aromatic radical such as alkyl, hydroxyalkyl, alkenyl, hydroxyalkenyl, aryl, alkylaryl, hydroxyalkyl, alkenyl, hydroxyalkenyl, aryl, alkylaryl, hydroxyalkylaryl, arylalkyl, alkenylaryl or arylalkenyl) containing from 6 to 30 (preferably from 8 to 18 and more preferably from 9 to 13) carbon atoms; R′ is a divalent hydrocarbon radical containing from 2 to 4 carbon atoms such as ethylene, propylene or butylene (most preferably the unit $(R'O)_y$ represents repeating units of ethylene oxide, propylene oxide and/or random or block combinations thereof); y is a number having an average value of from 0 to 12; Z represents a moiety derived from a reducing saccharide containing 5 or 6 carbon atoms (most preferably a glucose unit); and x is a number having an average value of from 1 to 10 (preferably from 1.5 to 10 and more preferably from 1.5 to 5).

Glycoside surfactants suitable for use herein also include those of the formula above in which one or more of the normally free (i.e., unreacted hydroxyl groups of the saccharide moiety, Z, have been alkoxylated; preferably, ethoxylated or propoxylated) so as to attach one or more pendant alkoxy or poly (alkoxy) groups in place thereof. In such event, the amount of alkylene oxide (e.g., ethylene oxide or propylene oxide) employed will typically range from 1 to 20 (preferably from 3 to 10) moles thereof per mole of saccharide moiety within the formula glycoside material.

In glycosides of the formula above, the $RO(R'O)_y$ group is generally bonded or attached to the number 1 carbon atom of the saccharide moiety, Z. Accordingly, the free hydroxyls available for alkoxylation are typically those in the number 2, 3, 4 and 6 positions in 6-carbon atom saccharides and those in the number 2, 3, 4 positions in the 5-carbon atom saccharides species. Typically, the number 2 position hydroxyls in the 5-carbon saccharides, and the number 2 and 6 position hydroxyls in 6-carbon saccharides, are substantially more reactive or susceptible to alkoxylation than those in the number 3 and 4 positions. Accordingly, alkoxylation will usually occur in the former locations in preference to the latter.

Glycoside surfactants especially preferred for use herein include those of the formula above wherein R is an alkyl group containing from 8 to 18 (especially from 9 to 13) carbon atoms; y is zero; Z is glucose or a moiety derived therefrom; and x has an average value of from 1.5 to 5 (especially from 1.5 to 3). It is to be noted that by use of alkyl polyglycosides, there often is no need for any hydrotropes such as urea, ethanol or $C_1$-$C_3$ alkylbenzene sulfonates.

Glycoside surfactants of particular interest for use in the practice of the present invention preferably have a hydrophilic-lipophilic balance (HLB) in the range of from 10 to 18 and most preferably in the range of from 12 to 14.

Within the compositions of the present claim, alkyl polyglycosides will be present in amounts ranging from 0.01 to 20% by weight, preferably from 0.5 to 10%, optimally between 1 and 2%.

Commercially, alkyl polyglycosides are available from the Horizon Chemical Company, a subsidiary of the A. E. Staley Manufacturing Company. These materials are sold under the trademark APG. Particularly preferred materials in this category are APG 23-3 and APG 91-3 which are $C_{12}$-$C_{13}$ and $C_9$-$C_{11}$ alcohol glycoside derivatives, respectively, having about three moles of glycosylation.

Defoaming of the wash may be accomplished by the presence of any of a number of commerically available defoaming agents. These agents may be of the general type of slightly soluble alkyl carboxylates, alkyl phosphates, hydrocarbon waxes, hydrophobic silicas, silicone defoamers, or many others. In addition to being an effective defoamer, the species must be stable to hypochlorite. The defoamer will optionally be present in the composition from 0.05% to 5%, preferably from 0.1 to 1%, and most preferably from 0.1 to 0.5% by weight of the composition.

Amounts of water present in the liquid compositions should neither be so high as to produce unduly low viscosity and fluidity, nor so low as to produce unduly high viscosity and low flowability, thixotropic properties in either case being diminished or destroyed. Water will generally be present in an amount ranging from 25 to 80%, preferably from 45 to 75%, optimally from 55 to 65% by weight of the composition.

An alkali metal hydroxide may be used as an alkaline source and as a means to boost the pH to stabilize hypochlorite. Although small amounts of sodium hydroxide may be utilized, this material is desirably excluded in favor of potassium hydroxide. The potassium hydroxide may be added in the form of an aqueous liquor or as a solid. Amounts of potassium hydroxide will range from 0.1 to 10%, preferably 0.5 to 5%, and optimally 1 to 2% by weight of the composition.

Minor amounts of various other adjuvants may be present in the gel composition. Thus, the compositions may include perfumes, flow control agents, soil suspending agents, antiredeposition agents, anti-tarnish agents, enzymes and other functional additives.

Liquid products tend to have more serious problems with component compatibility than occur with powder products. For instance, chlorine releasing bleaches, normally found in powdered formulations, oxidatively attack many co-components found in liquid automatic dishwashing detergent compositions. Oxidation sensitive components include perfumes, surfactants and dyes.

In a further aspect, this invention provides a cleaning composition comprising

( i) a clear gel as described above; and

(ii) opaque particles of an active material uniformly dispersed and suspended within said gel, said active material being surrounded by a protective substance, the ratio of said active material to protective substance ranging from 1:100 to 100:1 and said clear gel to said opaque particles being in a ratio of from 500:1 to 5:1.

Opaque particles may comprise an active material and one or more protective substances. The protective substances may be one or more encapsulating layers surrounding a core of active material. Alternatively, the active material may be embedded in a matrix of the protective substance. Agglomeration processing usually gives rise to the aforementioned active embedded matrix. In either situation, the ratio of total active material to total protective substance will range from 1:100 to 100:1, preferably from 1:10 to 15:1, optimally 1:1 to 6:1.

The weight ratio of transparent gel formulation to opaque particles will range from 500:1 to 5:1, preferably from 100:1 to 10:1, optimally about 20:1.

A wide variety of protective substances may be utilized and the substances will vary depending upon the active material protected and the eventual application of the cleaning composition. A protective substance may be defined as one that is non-reactive with the active material and prevents the active material from adversely interacting with the gel components and vice versa under storage conditions. Protective substances may include inorganic salts, hydrocarbon and vegetable waxes, organic esters, soaps, homo and copolymers, long chain fatty acids, polyalkoxylates, polyglycolates, organic amides and mixtures thereof. A characteristic of the foregoing substances is that the melting point must be between 25°C and 200°C, preferably between 35°C and 100°C. Among the inorganic protective substances that may be included are the glassy phosphates. Inorganic salts may also serve as a diluent protective substance intimately mixed or agglomerated with the active material to form a matrix, the total matrix being covered by a soap, homopolymer, copolymer, wax, or other organic surface coating.

A wide variety of homopolymers and copolymers are suitable as the protective substance. Illustrative homopolymers may be polyacrylates, polymethacrylates, polyethylene, polypropylene, polyoxypropylene, polyvinyl acetate and polyvinyl alcohol.

Illustrative copolymers may be those formed from styrene, acrylic acid, methacrylic acid, vinyl acetate, crotonic acid, vinyl neodecanoate and butenoic acid. Exemples of carboxylate type copolymers are the styrene/alkyl acrylate and partially esterified polyacrylic and polymethacrylic salts and free acid forms. Among the foregoing materials are poly(butyl methacrylate), poly(methyl acrylate), poly(methyl methacrylate), poly(acrylic acid/$C_1$-$C_{20}$ alkyl methacrylate), poly(methacrylic acid/$C_1$-$C_{20}$ alkyl acrylate), poly(acrylic acid/$C_1$-$C_{20}$ alkyl acrylate) and poly(methacrylic acid/$C_1$-$C_{20}$ alkyl methacrylate). These copolymers may be prepared by polymerization of the respective monomers by traditional oil-in-water or water-in-oil emulsion polymerization techniques. Alternatively, a pseudo latex may be prepared by esterification of preformed polymer with $C_1$-$C_{20}$ alkanol.

It is to be understood that the terms homopolymer and copolymer are each a sub-category of "polymer". Moreover, the term copolymer includes polymers fashioned from 2 to 6 different monomers in block or random linkage.

Active materials may include chlorine and oxygen bleaches, bleach precursors, enzymes, fabric softeners, surfactants, perfumes and mixtures thereof.

When the active material is a oxidizing material, it may be a chlorine or bromine releasing agent or a peroxygen compound. Among suitable reactive chlorine or bromine oxidizing materials are the heterocyclic N-bromo and N-chloro imides mentioned above.

Dry, particulate, water-soluble anhydrous inorganic salts are likewise suitable for use herein such as lithium, sodium or calcium hypochlorite and hypobromite. Chlorinated trisodium phosphate is another active material.

Organic peroxy acids may be utilized as the active material within the opaque particle. The peroxy acids usable in the present invention are solid and, preferably, substantially water-insoluble compounds. By "substantially water-insoluble" is meant herein a water-solubility of less than about 1% by weight at ambient temperature. In general, peroxy acids containing at least 7 carbon atoms are sufficiently insoluble in water for use herein.

Typical monoperoxy acids useful herein include alkyl peroxy acids, alkenyl peroxy acids and aryl peroxy acids such as:

( i) peroxybenzoic acid and ring-substituted peroxybenzoic acids, e.g. peroxy-α-naphthoic acid

( ii) aliphatic and substituted aliphatic monoperoxy acids, e.g. peroxylauric acid and peroxystearic acid.

Typical diperoxy acids useful herein include alkyl diperoxy acids, alkenyl diperoxy acids and aryldiperoxy acids, such as:

(iii) 1,12-diperoxydodecanedioic acid

( iv) 1,9-diperoxyazelaic acid

( v) diperoxybrassylic acid; diperoxysebacic acid and diperoxyisophthalic acid

( vi) 2-decyldiperoxybutane-1,4-dioic acid.

Inorganic peroxygen generating compounds may also be suitable as particles for coating. Examples of these materials are salts of monopersulfate, perborate monohydrate, perborate tetrahydrate, and percarbonate.

Solid bleach precursors or activators may also be usefully coated by the process. Illustrative of organic precursors are N,N,N′,N′-tetraacetylethylene diamine (TAED), benzoyloxybenzene sulfonate and sodium nonanoyloxybenzene sulfonate. Inorganic bleach catalysts such as manganese salts or manganese ions adsorbed onto aluminosilicate supporting substrates such as zeolites could also benefit from this invention. The manganese catalysts may be prepared according to the method primarily described in U.S. Patent 4,536,183 (Namnath). Other catalysts of this type are more fully described in U.S. Patent 4,601,845 (Namnath), U.S. Patent 4,626,373 (Finch et al.) and EP-A-0 237 111.

An especially preferred catalyst for promoting peroxygen bleaching is the complex of manganese (III) and a multidentate ligand supplied by a complexing agent, preferably a hydroxycarboxylic acid containing at least 5 carbon atoms and the salts, lactones, acid esters, ethers and boric esters thereof. Illustrative of such complexes is manganese (III) gluconate.

Although the gels of this invention have been specifically designed for automatic dishwashing compositions and the foregoing specification has detailed such formulation products, it must be emphasized that the base gel structure can be utilized for other purposes. Thus, it is envisioned that the gel composition of this invention may be useful in products such as fabric washing formulations, hand dishwashing liquids, toilet bowl scrubs, pot/pan cleaners, fabric softeners, denture cleaners and even shampoos.

The following examples will more fully illustrate the embodiments of this invention. All parts, percentages and proportions referred to herein and in the appended claims are by weight unless otherwise indicated.

## EXAMPLES 1-4

These Examples were formulated to investigate changes in the concentration of an aluminum containing material, Catapal® D alumina.

| Component | Examples 1 | 2 | 3 | 4 |
|---|---|---|---|---|
| Tetrapotassium pyrophosphate | 19.0 | 19.0 | 19.0 | 19.0 |
| Britesil® H20 (sodium silicate) | 7.5 | 7.5 | 7.5 | 7.5 |
| Potassium carbonate | 6.0 | 6.0 | 6.0 | 6.0 |
| Sodium tripolyphosphate | 1.0 | 1.0 | 1.0 | 1.0 |
| Potassium hydroxide | 1.0 | 1.0 | 1.0 | 1.0 |
| Carbopol 941® | 1.0 | 1.0 | 1.0 | 1.0 |
| Catapal® D Alumina | - | 0.05 | 0.1 | 0.2 |
| Water | to 100 | to 100 | to 100 | to 100 |
| Cup retention (viscosity) | | | | |
| at 5 $sec^{-1}$ shear (cps) | 1343 | 3571 | 6193 | 6493 |
| at 21 $sec^{-1}$ shear (cps) | 711 | 996 | 1956 | 2223 |

Example 1 is a formulation containing Carbopol 941® and the structuring chelants, potassium carbonate and potassium pyrophosphate. Alumina was absent from this formulation. Pourability of the Examples was evaluated by measuring the viscosity under high shear conditions (i.e. 21 $sec^{-1}$). Pourability is considered adequate if the value under high shear at 25 °C is no greater than 5,000 cps. On the other hand, the "at rest" or cup retention viscosity property was measured at low shear (5 $sec^{-1}$) at 25 °C. Here a value no lower than 1,500, preferably 3,000 cps is needed.

Under high shear, Example 1 was measured to have a value of 711 cps which indicated satisfactory pourability. However, the "at rest" viscosity value of 1343 cps was very poor. The formula of Example 1 would not adequately be retained by a dishwasher dispenser cup. By contrast, Example 2 had an adequate pourability of 996 cps and a very substantial viscosity of 3,571 cps when "at rest" in the dispenser cup. It is to be noted when using Catapal D Alumina, a plateau in the "at rest" or storage viscosity occurs at about 0.1 wt.%.

EXAMPLE 5

Elastic rebound or recoil properties are discussed in this Example. Viscoelasticity properties were measured using a Carrimed Control Stress Rheometer type 5010 operated in the Creep Mode. A cone-and-plate geometry was employed. Cone radius was 3 centimeters and cone angle was 2°. In the Creep Mode, a constant stress is applied to the sample during which sample deformation is traced over a period of time. This deformation typically has two components for a viscoelastic fluid. The viscous component increases linearly with time while the viscoelastic component rises at a rate which decreases with time, eventually reaching the steady state value. The steady state viscoelastic deformation can be used to define the steady state compliance $J_e°$. Tabulated below are the results of $J_e°$ measurements on a series of aqueous liquids or gels having various thixotropic components.

| Sample | Component | $J_e°$ |
|---|---|---|
| 1 | Base Formula*<br>1% Carbopol 941®<br>0.1 Catapal D Alumina | 0.038 |
| 2 | Base Formula*<br>1% Carbopol 941®<br>0.1% Catapal D Alumina<br>19% Tetrapotassium Pyrophosphate | 0.025 |
| 3 | Base Formula*<br>1% Linear Sodium Polyacrylate<br>0.1% Catapal D Alumina<br>19% Tetrapotassium Pyrophosphate | 0.000 |
| 4 | Commercial Clay-Based Automatic Dishwashing Liquid | 0.0025 |

* Base Formula: 7.5% Britesil H2O, 1% Potassium Hydroxide and water.

From the results tabulated above, it is evident that non-crosslinked linear sodium polyacrylate in the context of Catapal D Alumina and tetrapotassium pyrophosphate does not possess rebound as is the case with the formula using Carbopol 941®. Cross-linking is thus quite important with respect to the polyacrylate polymer. Commercial clay-based automatic dishwashing liquids or gels also do not have any significant amount of rebound as is apparent from the $J_e°$ values given above.

EXAMPLES 6-9

Illustrated herein is the effect of varying the concentration of Carbopol 941®. The "at rest" or storage viscosity substantially increases around 0.7% Carbopol 941® to achieve an improved thickness. Although the pour viscosity under shear also increases as polymer concentration increases, this viscosity still remains within an acceptable level.

| Component | Examples 6 | 7 | 8 | 9 |
|---|---|---|---|---|
| Tetrapotassium pyrophosphate | 19.0 | 19.0 | 19.0 | 19.0 |
| Britesil H20 (sodium silicate) | 7.5 | 7.5 | 7.5 | 7.5 |
| Potassium carbonate | 6.0 | 6.0 | 6.0 | 6.0 |
| Sodium tripolyphosphate | 1.0 | 1.0 | 1.0 | 1.0 |
| Potassium hydroxide | 1.0 | 1.0 | 1.0 | 1.0 |
| Catapal® D Alumina | 0.1 | 0.1 | 0.1 | 0.1 |
| Carbopol 941® | 0.4 | 0.6 | 0.8 | 1.0 |
| Water | to 100 | to 100 | to 100 | to 100 |
| Cup retention (viscosity) | | | | |
| at 5 $sec^{-1}$ shear (cps) | 744 | 1041 | 4315 | 6343 |
| at 21 $sec^{-1}$ shear (cps) | 265 | 531 | 2373 | 2089 |

EXAMPLE 10 (not within the scope of the invention)

This Example investigates the effect of varying levels of Laponite XLS® upon clarity of the gel. A series of compositions were prepared having the following basic composition.

| Base Composition | |
|---|---|
| Component | Weight % |
| Tetrapotassium Pyrophosphate | 19.0 |
| Britesil H2O (sodium silicate) | 7.5 |
| Potassium Carbonate | 6.0 |
| Sodium Tripolyphosphate | 1.0 |
| Potassium Hydroxide | 1.0 |
| Catapal® D Alumina | 0.1 |
| Laponite XLS® | as specified below |
| Water | to 100 |

Varying levels of Laponite XLS® were incorporated into these compositions and the clarity of each composition was noted as follows:

| Laponite XLS® (Weight %) | Appearance of Composition |
|---|---|
| 0 | Clear |
| 0.02 | Clear |
| 0.05 | Clear |
| 0.1 | Translucent |
| 0.2 | Almost Opaque |

Absent Laponite XLS®, the compositions do not exhibit proper viscosity and $J_e°$ values providing a recoil gel, but the composition has a clear appearance. Addition of only 0.02% Laponite XLS® to the base composition allows formation of a recoil gel which still retains a clear appearance. Similarly, increasing the level of the clay to 0.05% maintains clarity. Upon reaching 0.1% clay the formation becomes translucent. An almost opaque product occurs at a level of 0.2% clay. Therefore, under circumstances where both clarity and optimum rheology are desired, there is a narrow range of concentration for dosing the Laponite XLS®.

EXAMPLE 11

This Example investigates the effect of various polymeric thickeners on phase stability and clarity of the gel. A series of compositions were prepared having the following basic composition.

| Base Composition | |
|---|---|
| Component | Weight % |
| Tetrapotassium Pyrophosphate | 19.0 |
| Britesil H2O (sodium silicate) | 7.5 |
| Potassium Carbonate | 6.0 |
| Sodium Tripolyphosphate | 1.0 |
| Potassium Hydroxide | 1.0 |
| Catapal® D Alumina | 0.1 |
| Polymeric Thickener | as specified below |
| Water | to 100 |

The series of compositions incorporated the polymers as set out in the following table:

| Polymeric Thickener | Polymer Identification | Weight % | Stability | % Transmittance |
|---|---|---|---|---|
| Hercules CMC 7H4F® | Sodium Carboxymethyl Cellulose | 1.0 | Unacceptable | nil |
| Alco EXP 1098-5® | Hydrophobically Modified Polyacrylate | 1.2 | Unacceptable | nil |
| Alcogum SL-60® | Polyacrylic Acid Emulsion | 1.0 | Unacceptable | nil |
| Alcosperse 175® | Polyacrylic Acid (m.w. 20,000) | 1.0 | Unacceptable | nil |
| National Starch Polymer | Polyacrylic Acid (m.w. 5000) | 1.0 | Unacceptable | nil |
| Carbopol 907® | Cross-Linked Polyacrylic Acid (m.w. 450,000) | 2.4 | Borderline | 10 |
| Carbopol 934® | Cross-Linked Polyacrylic Acid (m.w. 3,000,000) | 1.0 | Borderline | 11 |
| Carbopol 940® | Cross-Linked Polyacrylic Acid (m.w. 4,000,000) | 1.0 | Borderline | -- |
| Carbopol 941® | Cross-Linked Polyacrylic Acid (m.w. 1,250,000) | 1.0 | Excellent | 60 |

All the non-crosslinked polymers listed in the table either precipitated in some way or phase separated from the electrolyte solution. All were non-homogeneous. Fully clear gels were also not obtainable. Carbopol 907®, 934® and 940® were not as clear as Carbopol 941® and were borderline with respect to phase stability.

EXAMPLE 12

Herein is presented the investigation of various co-structurants and their effect upon clarity of the gel. Co-structuring salts as listed in the following table were incorporated into a base composition which was the

same as for Example 15 but included 1% of Carbopol 941®.

| Co-Structurant | Viscosity (cps) | | Concentration Weight % | Fresh Appearance |
|---|---|---|---|---|
| | 5 $sec^{-1}$ | 21 $sec^{-1}$ | | |
| Catapal D Alumina (pseudoboehmite) | 7,942 | 3,118 | 0.1 | Clear |
| $MgSO_4$ | 5,450 | 1,351 | 0.236 | Clear |
| $Cr_2(SO_4)_3 \cdot 9H_2O$ | 11,939 | 3,407 | 0.437 | Opaque |
| $Cr_2O_3$ | 7,691 | 2,809 | 0.149 | Opaque |
| $Fe_2O_3$ | 4,180 | 1,692 | 0.156 | Opaque |
| $Fe_2(SO_4)_3 \cdot 9H_2O$ | 6,019 | 2,207 | 0.553 | Opaque |
| $CaCl_2 \cdot 2H_2O$ | 5,751 | 1,438 | 0.288 | Opaque |
| $CuSO_4 \cdot 5H_2O$ | 8,652 | 2,505 | 0.313 | Translucent |
| $ZnSO_4 \cdot 7H_2O$ | 7,524 | 1,739 | 0.563 | Opaque |
| $K_2SO_4$ | 2,082 | 752 | 0.17 | Transparent |

With the exception of Catapal® D Alumina, all the co-structurants listed above formed precipitate over time. Also, none of the above salts gave the degree of clarity provided by alumina.

EXAMPLE 13

The effects of various chelant/electrolyte combinations have also been investigated. A series of compositions were prepared having the following basic composition.

| Base Composition | |
|---|---|
| Component | Grams |
| Water | 34.5 |
| Potassium Hydroxide | 1.0 |
| Catapal D Alumina | 0.1 |
| Chelant/Electrolyte | as specified below |
| Carbopol 941® | 1.0 |

Various sodium and potassium compounds were incorporated into these compositions as chelant/electrolyte. Viscosities were measured, as for Example 1, and results are given in the following table:

| Chelant/Electrolyte | Grams | Viscosity (cps) | |
|---|---|---|---|
| | | 5 $sec^{-1}$ | 21 $sec^{-1}$ |
| Potassium nitrate | 20.0 | 3750 | 1607 |
| Potassium sulfate | 10.0 | 4875 | 1518 |
| Potassium tripolyphosphate | 15.0 | 3750 | 1250 |
| Sodium citrate | 10.0 | 1500 | 625 |
| Tetrasodium EDTA | 10.0 | 1012 | 919 |
| Dequest 2066® | 15.0 | 2625 | 1071 |
| Potassium chloride | 15.0 | 1275 | 677 |
| Potassium carbonate | 15.0 | 13125 | 4464 |
| Tetrapotassium pyrophosphate | 5.0 | 3348 | 1275 |
| Tetrapotassium pyrophosphate | 10.0 | 5691 | 1594 |
| Tetrapotassium pyrophosphate | 15.0 | 6026 | 1913 |
| Tetrapotassium pyrophosphate | 19.0 | 15750 | 5536 |

From the table, it is evident that potassium carbonate has a substantially greater viscosity increasing effect than an equivalent weight of potassium chloride, potassium nitrate or potassium tripolyphosphate. Tetrapotassium pyrophosphate is also seen to have a very substantial viscosity building property.

EXAMPLES 14-17

Illustrated here is the effect of nonionic surfactants upon glass cleaning performance. Ten (10) dinner plates and ten (10) clean glass tumblers were placed in a Kenmore dishwasher. Forty (40) grams of a 4:1 mixture of margarine and powdered milk were placed in the dishwasher. Plates and glasses were then washed with test liquid or control products. Dishwasher dispenser cups were filled with test liquid or control product at equal volume (approximately 40 grams of granular control versus 60 grams of test liquid). After each cycle, glasses were visually inspected and then placed in another machine. Each glass was numerically rated for spotting and filming on a scale of 0 to 4 (0 = best; 4 = worst). Values for each glass in each of four runs were averaged together for an overall rating.

The base composition of Example 11 was utilized and further included 1% Carbopol 940®, 1.75% sodium dichloroisocyanurate, and 2% surfactant. Examples 14 and 16 incorporated Poly-Tergent SLF-18® as the nonionic surfactant. Example 16, unlike Example 14 did not contain sodium dichloroisocyanurate. Example 15 utilized AKYPO® LA 294, a nonionic surfactant sold by BASF-Wyandotte Corp. and structurally identified as a phenol ethoxylated with 3 moles ethylene oxide and end-capped with sodium acetate. Example 17 contained only the base composition without surfactant. The control was a commercial opaque thixotropic liquid formulated with a clay thickener.

| Performance | | | |
|---|---|---|---|
| Example | Surfactant | Spotting | Filming |
| 14 | Poly-Tergent SLF-18® | 0.25 | 0.75 |
| 15 | AKYPO LA 294® | 2.8 | 1.9 |
| 16 | Poly-Tergent SLF-18® | 2.2 | 0.7 |
| 17 | None | 1.2 | 1.8 |
| Control (Commercial ADD Liquid) | -- | 2.3 | 1.3 |
| Control (Commercial ADD Powder) | -- | 0.4 | 0.5 |

From the performance table, it is seen that Example 18 with Poly-Tergent SLF-18® provided spotting and filming results superior to that of other liquids tested and to the control commercial liquid. The formulation was also performance competitive with a typical commercial powder formulation.

EXAMPLES 18-27

Gel formulations having the viscosity and $J_e°$ values of the present invention are outlined in the following Table I. These formulations vary in the type and amount of surfactant utilized, and also in the antifoam present.

**TABLE I**

| Component | 18 | 19 | 20 | 21 | 22 | 23 | 24 | 25 | 26 | 27 |
|---|---|---|---|---|---|---|---|---|---|---|
| | Example No: | | | | | | | | | |
| Water | 55.4 | 55.9 | 55.9 | 55.9 | 56.4 | 56.4 | 55.4 | 56.4 | 55.9 | 55.9 |
| Potassium hydroxide | 1.0 | 1.0 | 1.0 | 1.0 | 1.0 | 1.0 | 1.0 | 1.0 | 1.0 | 1.0 |
| Tetrapotassium pyrophosphate | 19.0 | 19.0 | 19.0 | 19.0 | 19.0 | 19.0 | 19.0 | 19.0 | 19.0 | 19.0 |
| Catapal D alumina | 0.1 | 0.1 | 0.1 | 0.1 | 0.1 | 0.1 | 0.1 | 0.1 | 0.1 | 0.1 |
| Sodium tripolyphosphate | 1.0 | 1.0 | 1.0 | 1.0 | 1.0 | 1.0 | 1.0 | 1.0 | 1.0 | 1.0 |
| Carbopol 941 | 1.0 | 1.0 | 1.0 | 1.0 | 1.0 | 1.0 | 1.0 | 1.0 | 1.0 | 1.0 |
| Britesil H20 | 7.5 | 7.5 | 7.5 | 7.5 | 7.5 | 7.5 | 7.5 | 7.5 | 7.5 | 7.5 |
| Potassium carbonate | 6.0 | 6.0 | 6.0 | 6.0 | 6.0 | 6.0 | 6.0 | 6.0 | 6.0 | 6.0 |
| APG 23-3 | 2.0 | 1.0 | 1.0 | - | - | - | - | - | - | - |
| APG 91-3 | - | - | - | 1.0 | - | - | - | - | - | - |
| APG 9HDP | - | - | - | - | 1.0 | - | - | - | - | - |
| APG 14HDP | - | - | - | - | - | 1.0 | - | - | - | - |
| Polytergent SLF-18 | - | - | - | - | - | - | 2.0 | - | - | - |
| Dowfax 2A1 | - | - | - | - | - | - | - | 1.0 | 1.0 | - |
| SAG 1000 | - | 0.5 | - | 0.5 | - | - | - | - | - | - |
| DB 100 | - | - | 0.5 | - | - | - | - | - | 0.5 | - |
| APG 35-H | - | - | - | - | - | - | - | - | - | 1.0 |

Clarity was determined by a measurement of percent transmittance; the higher the transmittance value, the clearer the gel. Results are set out in Table II below.

From Table II, it is evident that all the various alkyl polyglycosides have a transmittance in excess of 20% which is considered transparent within the context of this invention. Polytergent SLF-18®, when incorporated into the gel base formulation at only 2% caused a severe decrease in percent transmittance. See Example 24. By contrast, incorporation of 2% APG 23-3 caused no decrease, and in fact, provided a slight increase in percent transmittance relative to the gel base.

TABLE II

| Example | % Transmittance |
|---|---|
| Water | 100.0 |
| Gel base (excluding surfactant and defoamer) | 75.9 |
| 18 | 77.6 |
| 19 | 24.7 |
| 20 | 27.8 |
| 21 | 52.8 |
| 22 | 60.9 |
| 23 | 64.4 |
| 24 | 1.4 |
| 25 | 77.6 |
| 27 | 64.2 |

EXAMPLE 28

Foam is of particular concern with automatic dishwashing compositions. Accordingly, several of the formulations of Table I were measured for their propensity to cause foam. Low foam values are the desired objective.

Measurements of relative foam height levels for various compositions were made using a thermally jacketed foam meter and two types of food soils.

A formulated gel composition is prepared and 2.00 gm. of this is added to 500 ml. of tap water at 45 °C., so that a level approximately equal to a dosage of 40 gm. is met. A high shear is then applied at the base of the foam meter by means of a commercial Waring blender for one minute. The blender is then shut off and the wash liquor allowed to settle for one minute before a reading is taken.

Measurements that include soils are also performed whereby the wash liquor is doped with either 2.0 gm. of a butter/dry milk mix, or 10 ml. of an egg yolk solution prepared from a premix of one egg yolk in 100 ml. of water.

Scores are then reported as a measured foam height of a particular composition with or without the presence of a soil, and the difference (D) of a soil-only foam height versus the same soil with a given gel formulation. Results are given in Table III below.

TABLE III

| Foam Height Measurements | | | | |
|---|---|---|---|---|
| Soil type | Example | No Soil | Soil | D |
| Egg yolk | - | - | 5 | - |
| | 18 | 5 | 10 | 5 |
| | 19 | 2 | 5 | 0 |
| | 20 | 2 | 4 | -1 |
| | 25 | 14 | 9 | 4 |
| | 26 | 5 | 7 | 2 |
| Butter/dry milk | - | - | 7 | - |
| | 18 | 5 | 11 | 4 |
| | 19 | 0 | 4 | -3 |
| | 20 | 2 | 5 | -2 |
| | 24 | 0 | 4 | -3 |
| | 25 | 14 | 11 | 4 |
| | 26 | 15 | 8 | 1 |

EXAMPLE 29

Three of the compositions of Table I were tested for glass cleaning performance by the procedure of Examples 14 to 17. Results are given in Table IV below.

TABLE IV

| Cleaning Performance | | |
|---|---|---|
| Example | Average Spotting | Average Filming |
| 18 | 2.21 | 0.68 |
| 19 | 1.62 | 2.07 |
| 24 | 1.05 | 2.52 |

EXAMPLE 30

The formulation of Example 3 was used for formulations which additionally contained chlorine bleach. In a control formulation there was incorporated an amount of sodium hypochlorite sufficient to provide 1% available chlorine to the base formulation. At room temperature, the product began to degrade after about two weeks. A decreased viscosity and stringiness resulted and the formula no longer was able to remain in a dishwasher dispenser cup. When storage stability was evaluated at 40 °C, degradation was even faster and more pronounced.

Degradation is believed to arise from attack of the Carbopol 941® by the bleach. In a test formulation intended to overcome the incompatibility problem, the bleach was encapsulated as follows. Anhydrous sodium dichloroisocyanurate was coated with a polyethylene/wax protective composition to obtain spherical particles of uniform size and appearance. These capsules had the following composition:

Encapsulated Chlorine Bleach

Active Material Mixture (Fill): 72.7%

Anhydrous sodium dichloroisocyanurate (ACL 60®)

Protective Substance (Shell): 27.3%

Polyethylene/Paraffin Wax Combination
Percent Available Chlorine (initial) = 16.9%
Particle Size: 595 to 1000 microns

Encapsulates were prepared with the aid of an extrusion device as described in Chemical Technology, October 1974, article by Goodwin and Somerville entitled "Macroencapsulation by Physical Methods". The extrusion device has a head with two nozzles and a concentric feed tube which enters the head through a seal arrangement. A rotating shaft is attached to the device so that the direction of rotation is around the vertical axis of the device. Shell and fill material are pumped separately through the feed tube into the head and to the nozzles which consist of concentric orifices. As the heat rotates, shell material flows through the outer orifice of the nozzle and fill material flows through the inner orifice of the nozzle. Thereby a rod of fill material is created which is surrounded by a sheath of shell material. This extruded rod of material eventually breaks into individual capsules which are then collected.

Encapsulated bleach particles in an amount approximately 7% by weight of the total composition, and sufficient to provide 1% available chlorine, were mixed with the base formulation to obtain a finished product. Calculation of percent available chlorine remaining in the finished product van determined by a standard Iodometric titration method. There was, however, one slight modification of the method involving use of a Waring blender to ensure complete release of chlorine from the capsules. Samples of the gel were stored at room temperature and analyzed for percent remaining available chlorine on a weekly basis. Table V below compares these results with respect to the control formulation containing unencapsulated sodium hypochlorite.

TABLE V

| Capsule Stability versus Unencapsulated Hypochlorite in Detergent Gel | | |
|---|---|---|
| Time (weeks) | % Available Chlorine | |
| | Gel with Capsules | Gel with Unencapsulated Hypochlorite |
| 0 | 1.18 | 1.20 |
| 1 | 1.20 | 0.08 |
| 2 | 1.15 | 0.02 |
| 3 | 1.10 | 0.0 |
| 4 | 1.05 | 0.0 |
| 5 | 0.97 | 0.0 |
| 6 | 0.97 | 0.0 |
| 8 | 0.85 | 0.0 |

Results in Table V indicate a significant improvement in chlorine stability over a system formulated with sodium hypochlorite. Visual examination of the samples stored at both room temperature and 40 °C showed that the encapsulate containing gel retained original rheology throughout the eight weeks of testing. Most importantly, there was no significant degradation of gel rheological properties during the test period. Gel clarity was also maintained.

EXAMPLE 31

Illustrated within this Example is an alternate method of preparing encapsulated bleach particles. Sodium dichloroisocyanurate dihydrate (Clearon CDB-56®) at a weight of 80 grams is charged to a lab scale fluid bed coating apparatus. The fluidized bed is warmed to 60 °C. A solution of styrene/maleic anhydride copolymer, half butyl ester, available commercially as SMA 1440® from Arco Chemical Company, is dispersed in an acetone-water solvent at about 10% concentration. The polymer solution is then atomized onto the fluidized CDB-56 particles for about two hours at a pump rate of about 2.5 ml/per minute. After all the polymer solution is exhausted, the capsules are then further fluidized for 15-30 minutes to remove residual solvent. Subsequent thereto, about 5 grams of paraffin wax dissolved in a volatile hydrocarbon solvent is added to the fluid bed to form an outer second coating.

The resultant capsules are then dispersed under low shear in the base formulation of Example 3 (as used for Example 30) to provide an automatic dishwashing composition.

EXAMPLE 32

The following illustrates an active material other than chlorine bleach and a still further type of encapsulation technology that may be employed within the context of the present invention.

An oxygen-releasing bleach, diperoxydodecanedioic acid (DPDA) in an amount of 800 grams is charged into a fluidized bed (Aeromatic or Glatt equipment). A water latex of a copolymer based on poly(methacrylic acid) at approximately 50% solids is atomized into the fluid bed at a controlled rate. The temperature of the fluid bed must be held within 10 °C lower to 20 °C higher than the glass transition temperature of the copolymer. As the water evaporates, a coating of copolymer surrounds the DPDA. Encapsulated bleach particles are then retrieved from the fluid bed apparatus.

A gel may then be prepared by mixing under low shear the base formulation of Example 3 (as used for Example 31) with the opaque encapsulated particles prepared according to the above method.

## Claims

**1.** A cleansing composition in gel form having a viscosity on a Haake Rotovisco RV-100 viscometer at 25 °C under 5 $sec^{-1}$ shear of from 1,000 to 20,000 cps and under 21 $sec^{-1}$ shear of from 200 to 5,000 cps, and a pH range from 11 to 13, characterized in that it comprises:
(i) from 0.1 to 10% by weight of a thickener that is a cross-linked polycarboxylic polymer; and
(ii) from 0.01 to 10% by weight of a trivalent metal containing material other than aluminosilicate, said composition having a steady state viscoelastic deformation
compliance $J_e°$ value greater than 0.01 $m^2$/Newton.

2. A composition according to claim 1, characterized in that it is clear and has a maximum transmittance of light through a sample 2 cm thick of at least 10%.

3. A composition according to claim 1 or 2, characterized in that the polycarboxylic polymer is a copolymer of an alpha-beta monoolefinically unsaturated lower aliphatic carboxylic acid cross-linked with a polyether of a polyol, and the molecular weight of the polymer thickener ranges from 500,000 up to 10,000,000.

4. A composition according to any one of claims 1 to 3, characterized in that the trivalent metal is aluminum.

5. A composition according to any one of claims 1 to 4, characterized in that it further comprises an effective amount of a clay so as to obtain said gel.

6. A composition according to claim 5, characterized in that the clay is a natural or synthetic hectorite, and is present in an amount from 0.005 to 0.1% by weight.

7. A composition according to any one of claims 1 to 6, further comprising from 1 to 60% by weight of a water-soluble structuring chelant selected from the salts of carbonate, pyrophosphate and mixtures thereof.

8. A composition according to any one of the preceding claims 1-7 further comprising from 0.1 to 10% by weight of a chlorine releasing oxidizing agent.

9. A composition according to any one of the preceding claims 1-8 further comprising from 0.1 to 25% by weight of a surfactant.

10. A composition according to claim 9 wherein said surfactant is a $C_6$-$C_{10}$ alkanol alkoxylated with a mixture of ethylene oxide and propylene oxide.

11. A composition according to claim 9 which comprises from 0.01 to 20% by weight of an alkyl polyglycoside.

12. A composition according to claim 11 wherein the alkyl polyglycoside has the formula:

$$RO(R'O)_y(Z)_x$$

wherein R is a monovalent organic radical containing from 6 to 30 carbon atoms; R' is a divalent hydrocarbon radical containing from 2 to 4 carbon atoms; y is a number having an average value of from 0 to 12; Z represents a moiety derived from a reducing saccharide containing 5 or 6 carbon atoms; and x is a number having an average value of from 1 to 10.

13. A composition according to any one of the preceding claims 1 and 3-12 further comprising opaque particles of an active material uniformly dispersed and suspended within said gel, said active material being surrounded by a protective substance, the weight ratio of said active material to protective substance ranging from 1:100 to 100:1 and said transparent gel to said opaque particles being in a weight ratio of from 500:1 to 5:1.

14. A composition according to claim 13 wherein the active materials are selected from the group consisting of chlorine and oxygen bleaches, bleach precursors, enzymes, fabric softeners, surfactants, perfumes, and mixtures thereof.

15. A composition according to claim 13 or claim 14 wherein the opaque particles have an average size which ranges from 100 microns up to 3,000 microns.

## Patentansprüche

1. Reinigungsmittel in Gelform mit einer Viskosität auf einem Haake Rotovisco RV-100-Viskosimeter bei 25 °C unter 5 $s^{-1}$ Scherwirkung von 1000 bis 20000 und cPs unter 21 $s^{-1}$ Scherwirkung von 200 bis 5000 cPs und einem pH-Bereich von 11 bis 13, **dadurch gekennzeichnet,** daß es umfaßt:
   (i) 0,1 bis 10 Gew.-% eines Verdickungsmittels, das ein vernetztes Polycarbonsäurepolymer darstellt; und
   (ii) 0,01 bis 10 Gew.-% eines von Aluminosilicat verschiedenen Materials, das ein dreiwertiges Metall enthält, wobei das Mittel einen viskoelastische Deformationsnachgiebigkeitswert $J_e$° im Steady-State von mehr als 0,01 $m^2$/Newton aufweist.

2. Mittel nach Anspruch 1, **dadurch gekennzeichnet,** daß es klar ist und eine maximale Durchlässigkeit für Licht durch eine 2 cm dicke Probe von mindestens 10 % aufweist.

3. Mittel nach Anspruch 1 oder 2, **dadurch gekennzeichnet,** daß das Polycarbonsäurepolymer ein Copolymer einer $\alpha,\beta$-monoolefinisch-ungesättigten niederaliphatischen Carbonsäure ist, vernetzt mit einem Polyether eines Polyols und das Molekulargewicht des polymeren Verdickungsmittels im Bereich von 500 000 bis 10 000 000 liegt.

4. Mittel nach einem der Ansprüche 1 bis 3, **dadurch gekennzeichnet,** daß das dreiwertige Metall Aluminium ist.

5. Mittel nach einem der Ansprüche 1 bis 4, **dadurch gekennzeichnet,** daß es zusätzlich eine wirksame Menge eines Tons enthält, so daß das Gel erhalten wird.

6. Mittel nach Anspruch 5, **dadurch gekennzeichnet,** daß der Ton ein natürlicher oder synthetischer Hectorit ist und in einer Menge von 0,005 bis 0,1 Gew.-% vorliegt.

7. Mittel nach einem der Ansprüche 1 bis 6, zusätzlich umfassend 1 bis 60 Gew.-% eines wasserlöslichen strukturierenden Chelators, ausgewählt aus den Salzen von Carbonat, Pyrophosphat und Gemischen davon.

8. Mittel nach einem der vorangehenden Ansprüche 1 bis 7, zusätzlich umfassend 0,1 bis 10 Gew.-% eines chlorfreisetzenden Oxidationsmittels.

9. Mittel nach einem der vorangehenden Ansprüche 1 bis 8, zusätzlich umfassend 0,1 bis 25 Gew.-% eines Tensids.

10. Mittel nach Anspruch 9, worin das Tensid ein $C_6$-$C_{10}$-Alkanol, alkoxyliert mit einem Gemisch von Ethylenoxid und Propylenoxid ist.

11. Mittel nach Anspruch 9, umfassend 0,01 bis 20 Gew.-% eines Alkylpolyglycosids.

12. Mittel nach Anspruch 11, worin das Alkylpolyglycosid die Formel:

    $RO(R'O)_y(Z)_x$

    aufweist, worin R einen einwertigen organischen Rest mit 6 bis 30 Kohlenstoffatomen darstellt; R' einen zweiwertigen Kohlenwasserstoffrest mit 2 bis 4 Kohlenstoffatomen bedeutet und y eine Zahl mit einem durchschnittlichen Wert von 0 bis 12 darstellt; Z einen Rest abgeleitet von einem reduzierenden Saccharid mit 5 oder 6 Kohlenstoffatomen wiedergibt; und x eine Zahl mit einem durchschnittlichen Wert von 1 bis 10 bedeutet.

13. Mittel nach einem der vorangehenden Ansprüche 1 und 3 bis 12, zusätzlich umfassend trübe Teilchen eines wirksamen Materials gleichförmig dispergiert und suspendiert in dem Gel, wobei das wirksame Material umhüllt ist durch eine schützende Substanz, das Gewichtsverhältnis des wirksamen Materials der schützenden Substanz im Bereich von 1:100 bis 100:1 liegt und das transparente Gel zu den trüben Teilchen im Gewichtsverhältnis von 500:1 bis 5:1 steht.

14. Mittel nach Anspruch 13, wobei die aktiven Materialien ausgewählt sind aus der Gruppe, bestehend aus Chlor und Sauerstoffbleichmitteln, Bleichmittelvorstufen, Enzymen, textilweichpflegenden Mitteln, Tensiden, Parfumes und Gemischen davon.

15. Mittel nach Anspruch 13 oder Anspruch 14, wobei die trüben Teilchen eine durchschnittliche Größe im Bereich von 100 $\mu$m bis zu 3000 $\mu$m aufweisen.

**Revendications**

1. Composition de nettoyage sous forme de gel ayant une viscosité mesurée sur un Viscosimètre Haake Rotovisco RV-100 à 25°C sous un cisaillement de 5 $sec^{-1}$ de 1 000 à 20 000 cpo et sous un cisaillement de 21 $sec^{-1}$ une viscosité d'environ 200 à 5 000 cpo, et un intervalle de pH compris entre 11 et 13, caractérisée en ce qu'elle comprend (i) entre 0,1 et 10 % en poids d'un épaississant qui est un polymère polycarboxylique réticulé; et (ii) entre 0,01 et 10 % en poids d'un matériau contenant un métal trivalent différent d'un aluminosilicate, ladite composition a un coefficient de déformation viscoélastique en régime permanent $J_e°$ supérieur à 0,01 $m^2$/neutron.

2. Composition selon la revendication 1, caractérisée en ce qu'elle est claire et a une transmission maximale de lumière à travers un échantillon de 2 cm d'épaisseur d'au moins 10 %.

3. Composition selon la revendication 1 ou 2, caractérisée en ce que le polymère carboxylique est un copolymère d'acide carboxylique aliphatique inférieur à insaturation alpha-bêta monooléfinique, réticulé avec un polyéther de polyol, et le poids moléculaire de l'épaississant polymère est compris entre 500 000 et 10 000 000.

4. Composition selon l'une quelconque des revendications 1 à 3 caractérisée en ce que le métal trivalent est l'aluminium.

5. Composition selon l'une quelconque des revendications 1 à 4, caractérisée en ce qu'elle comprend en outre une quantité efficace d'argile de façon à obtenir ledit gel.

6. Composition selon la revendication 5, caractérisée en ce que l'argile est une hectorite naturelle ou synthétique et qu'elle est présente à une concentration de 0,005 à 0,1 % en poids.

7. Composition selon l'une quelconque des revendications 1 à 6, qui comprend en outre de 1 à 60 % en poids d'agent chélatant structurant soluble dans l'eau choisi parmi les sels carbonate, pyrophosphate et leurs mélanges.

8. Composition selon l'une quelconque des revendications 1-7 précédentes qui comprend en outre de 0,1 à 10 % en poids d'un agent oxydant dégageant du chlore.

9. Composition selon l'une quelconque des revendications précédentes 1-8 qui comprend en outre 0,1 à 25 % en poids de tensioactif.

10. Composition selon la revendication 9 dans laquelle ledit agent tensioactif est un alcanol $C_6$-$C_{10}$ alcoxylé avec un mélange d'oxyde d'éthylène et d'oxyde de propylène.

11. Composition selon la revendication 9 qui comprend entre 0,01 et 20 % en poids d'un alkylpolyglycoside.

12. Composition selon la revendication 11 dans laquelle l'alkylpolyglycoside a la formule

$$RO(R'O)_y(Z)_x$$

dans laquelle R est un radical organique monovalent contenant entre 6 et 30 atomes de carbone; R' est un radical hydrocarbure divalent contenant de 2 à 4 atomes de carbone; y est un nombre ayant une valeur moyenne comprise entre 0 et 12; Z représente un radical dérivé d'un saccharide réducteur contenant 5 ou 6 atomes de carbone; et x est un nombre ayant une valeur moyenne comprise entre 1

et 10.

**13.** Composition selon l'une quelconque des revendications précédentes 1 et 3-12 qui comprend en outre des particules opaques d'un matériau actif uniformément dispersé et mis en suspension dans ledit gel, ledit matériau actif étant enrobé d'une substance protectrice, la proportion pondérale dudit matériau actif à la substance protectrice est comprise entre environ 1:100 et 100:1, et celle dudit gel transparent auxdites particules opaques est de 500:1 à 5:1.

**14.** Composition selon la revendication 13 dans laquelle les matériaux actifs sont sélectionnés dans le groupe constitué des agents de blanchiment au chlore et à l'oxygène, précurseurs de blanchiment, enzymes, adoucissants textiles, tensioactifs, parfums et leurs mélanges.

**15.** Composition selon la revendication 13 ou la revendication 14 dans laquelle les particules opaques ont une taille moyenne comprise entre 100 microns et 3 000 microns.